# EUROPÄISCHE PATENTSCHRIFT

(11) **EP 3 305 502 B1**
(45) Veröffentlichungstag und Bekanntmachung des Hinweises auf die Patenterteilung: **04.12.2024**
(21) Anmeldenummer: 16002181.2
(22) Anmeldetag: 10.10.2016
(51) Int. Cl.: B29C 48/08, B29C 48/74, B29C 48/92, G01N 33/44, G01N 21/89, G01N 11/04, G01N 21/85, G01N 21/896

(54) **VORRICHTUNG UND VERFAHREN ZUR ÜBERWACHUNG VON MATERIALEIGENSCHAFTEN VON KUNSTSTOFFEN**
DEVICE AND METHOD FOR MONITORING MATERIAL PROPERTIES OF PLASTICS
DISPOSITIF ET PROCÉDÉ DE SURVEILLANCE DE CARACTÉRISTIQUES DE MATIÈRES PLASTIQUES

(43) Veröffentlichungstag der Anmeldung: 11.04.2018
(73) Patentinhaber: Collin Lab & Pilot Solutions GmbH, 83558 Maitenbeth (DE)
(72) Erfinder: Kastner, Friedrich, Dl, 4710 Grieskirchen (AT)
(74) Vertreter: Burger, Hannes Alfred

(56) Entgegenhaltungen:
- WO-A1-2016/037205
- DE-A1- 3 826 095

## Beschreibung

Die Erfindung betrifft eine Vorrichtung zur Überwachung von Materialeigenschaften von Kunststoffen während des Produktionsverfahrens.

Verfahren und entsprechende Vorrichtungen zur Überprüfung und Messung der physikalischen Parameter und Materialeigenschaften von Kunststoffen sind bekannt.

So wird beispielsweise bei Kunststoffproduktion, insbesondere bei der Folienproduktion, ein Druckfiltertest zur Ermittlung der Dispergierung von Additiven oder Verunreinigungen eines Polymers, ein Rheometer zur Bestimmung der Viskositätseigenschaften, eine optische Folieninspektion, Farbmessungen, Nahinfrarotspektroskopie (NIR), Fourier-Transformations-Infrarotspektrometrie, Massenspektrometrie oder mechanische Festigkeitsmessungen, beispielsweise Zug-, Dehnungsmessungen, Kratzfestigkeit der Oberfläche und dergleichen) zur Bestimmung der Eigenschaften des Produkts eingesetzt.

Diese Untersuchungsmethoden sind zum großen Teil genormt.

WO 2016/037 205 A1 offenbart ein Verfahren und eine Vorrichtung zur modularen Materialanalyse für Kunststoffe umfassend mindestens zwei unterschiedliche Messgeräte, die mechanisch und/oder funktional miteinander verbunden sind.

DE 38 26 095 A1 offenbart eine Vorrichtung zur Entnahme von Schmelzproben, insbesondere aus kontinuierlich arbeitenden kunststoffverarbeitenden Anlagen.

In der Regel werden diese Untersuchungen nach Probeentnahme während es Produktionsprozesses bzw. nach der Herstellung des Produkts offline in verschiedenen Messvorrichtungen meist extern in Analyse- und Qualitätskontrolllabors durchgeführt.

Die entsprechenden Ergebnisse stehen dann meist erst nach Abschluss des Produktionsprozesses zur Verfügung, wodurch eine Reaktion auf Qualitätsabweichungen während des Produktionsprozesses nahezu unmöglich wird.

Dadurch erhöht sich die Menge an fehlerhaften Produkten, die den Qualitätsanforderungen nicht entsprechen. Dieser Ausschuss muss dann aussortiert und/oder nachbearbeitet werden.

Es ist daher vorteilhaft, die relevanten Eigenschaften des herzustellenden Produkts bzw. des Rohmaterials oder des Vormaterial während des Produktionsprozesses zu analysieren um rechtzeitig in den Produktionsprozess eingreifen zu können und damit die Menge an fertigem Produkt zu reduzieren, das nicht den Qualitätserfordemissen entspricht.

Aufgabe der Erfindung war es, eine Vorrichtung und ein Verfahren zur Qualitätsüberprüfung und Qualitätsbeurteilung für Kunststoffe bereitzustellen, die es erlaubt, inline, also bereits während des Produktionsprozesses bestimmte Parameter und Eigenschaften des Produkts und des Vorprodukts zu bestimmen um zeitnah auf Abweichungen vom Sollzustand reagieren zu können und damit die Herstellung fehlerhafter Produkte weitgehend zu vermeiden.

Die Erfindung löst diese Aufgabe durch die Vorrichtung bzw. das Verfahren der unabhängigen Ansprüche. Vorteilhafte Weiterbildungen sind in den abhängigen Ansprüchen dargestellt. Gegenstand der Erfindung ist daher eine Vorrichtung zur Inline-Qualitätsüberprüfung für Kunststoffe während des Produktionsprozesses umfassend mindestens zwei unterschiedliche Messgeräte ausgewählt aus der Gruppe Messgeräte zur Bestimmung der rheologischen Eigenschaften, Messgeräte zur Farbmessung, Messgeräte zur Detektion von Einschlüssen und Gelpartikeln und dergleichen, Messgeräte zur Beurteilung der optischen Eigenschaften und Messgeräte zur Bestimmung der mechanischen Parameter, dadurch gekennzeichnet, dass die Vorrichtung eine Einrichtung zur Entnahme von Kunststoffschmelze aus dem Produktionsextruder, eine Einrichtung zum Formen einer Flachfolie aus dieser Kunststoffschmelze und Einrichtungen zum Transport der Flachfolie zu den Messgeräten und zum Konfektionieren der Flachfolie aufweist, wobei die Vorrichtung über einen Adapter an einen Seitenstrang des Extruders gekoppelt ist und nach Öffnen eines Ventils die Entnahme der Kunststoffschmelze erfolgt, und die Entnahme der Kunststoffschmelze durch einen Verdrängungskörper, der im Schmelzstrom positioniert ist und um den die Schmelze zu einer Bohrung im Zylindergehäuse des Extruders geleitet wird, erfolgt.

Ein weiterer Gegenstand der Erfindung ist ein Verfahren zur Qualitätsüberprüfung für Kunststoffe durch eine Vorrichtung während des Produktionsprozesses, wobei die Vorrichtung über einen Adapter an einen Seitenstrang eines Produktionsextruder gekoppelt ist, wobei das Verfahren gekennzeichnet ist durch Öffnen eines Ventils, Entnehmen einer definierten Menge an Kunststoffschmelze aus dem Produktionsextruder durch einen Verdrängungskörper, der im Schmelzstrom positioniert ist und um den die Schmelze zu einer Bohrung im Zylindergehäuse des Extruders geleitet wird, Formen dieser Kunststoffschmelze zu einer Flachfolie, Transportieren der Flachfolie zu in der Vorrichtung situierten Messgeräten, und Untersuchen und Beurteilen der Flachfolie.

Durch die Entnahme der Schmelze inline direkt aus dem Produktionsextruder und die Prüfung der aus der Schmelze geformten Flachfolie noch während des Produktionsprozesses kann auf Qualitätsabweichungen unmittelbar und zeitnah reagiert werden und so unnötiger Ausschuss am Produkt vermieden werden.

Die erfindungsgemäße Vorrichtung weist eine Einrichtung zur Entnahme einer Probe der Kunststoffschmelze aus dem Produktionsextruder auf. Diese Einrichtung wird im einfachsten Fall durch einen Adapter, der im einfachsten Fall eine Bohrung aufweist, an der eine Schmelzeleitung zur erfindungsgemäßen Vorrichtung angebracht ist, oder durch eine Bohrung direkt im Zylindergehäuse des Austragsbereiches des Extruders realisiert

Mit diesen Lösungen nimmt man allerdings den Nachteil in Kauf, dass bedingt durch Randeffekte die Strömungsgeschwindigkeit am Rand sehr gering ist, und man daher Gefahr läuft eigentlich schon veränderte, abgebaute oder zeitlich nicht mehr relevante Proben mit dem Materialstrom zu korrelieren.

Um dies zu vermeiden, können erfindungsgemäß Adapter oder Zwischenstücke eingebaut werden, die sicherstellen, dass über den gesamten oder zumindest ab oder in der Mitte des Hauptstroms Schmelzeproben entnommen werden.

In einer einfachen Ausführung ist hier die Entnahmeöffnung einfach ein Rohrende, das in den Schmelzestrom gerichtet ist.

Es wird ein Verdrängungskörper in den Schmelzestrom eingebracht, welcher derartig ausgeführt ist, dass sich in diesem Verdrängungskörper eine Entnahmestelle für den Schmelzestrom befindet. Die Entnahmestelle kann dabei entweder eine Bohrung sein, die den Schmelzestrom innerhalb des Verdrängungskörpers aus den Extruder leitet, oder ein Schmelzekanal, der den Schmelzestrom entlang der Oberfläche des Verdrängungskörper ableitet.

In beiden Fällen ist es notwendig, dass die Entnahmestelle so ausgeformt ist, dass sich ein selbständiger Schmelzeaustrag auf Grund der im Extruder vorherrschenden Druck- und Strömungsverhältnisse ergibt. Die Ausführung als Schmelzekanal ist insofern vorteilhaft, als dass eine, repräsentativ radial über die im Zylinder des Extruders strömende Schmelzeprobe entnommen werden kann.

Eine einfache Ausführungsform eines solchen Verdrängungskörpers kann eine diagonal durch den Zylinder des Extruders verlaufende, strömungstechnisch optimierte Speiche mit einem an der Stirnseite angebrachten Schmelzekanal darstellen.

Ebenso können aber auch drei oder mehr Speichen sternförmig ausgeführt werden. In beiden Ausführungsformen ist es vorteilhaft wenn der Verdrängungskörper torpedoartig entgegen der Strömungsrichtung im Extruder positioniert ist.

Mit dem Adapter wird die erfindungsgemäße Vorrichtung an einen Seitenstrang des Extruders gekoppelt und nach Öffnen eines Ventils über eine Schmelzepumpe eine definierte Menge an Kunststoffschmelze aus dem Extruder entnommen. Dabei wird bereits mittels eines Rheometers, bestehend aus einer Messdüse mit definiertem Querschnitt, über eine bestimmte Messstrecke der Druckverlust gemessen. Aus dem Druckverlust kann die Viskosität der Schmelze bestimmt werden.

Aus der Schmelze wird anschließend mittels einer Breitschlitzdüse und einer Gießwalze eine Flachfolie zur Ermittlung weiterer Eigenschaften hergestellt. Hier ist besonders wichtig, dass die Folie homogene Eigenschaften (Dicke, Farbe, etc.) aufweist um später eine optimale Kontrolle zu gewährleisten. Daher ist eine optimale Kühlung aber auch die Beschaffenheit der Oberfläche der Gießwalze wesentlich.

Im einfachsten Fall ist die Oberfläche glänzend ausgeführt. Um die Ablösung von verschiedenen Materialen zu gewährleisten, kann die Walze mit Beschichtungen wie Glanzchrom, Antihaftschichten wie Teflon, oder anderen gängigen Antihaftschichten oder Kombinationen daraus hergestellt sein.

Um für spezielle Untersuchungen bessere Bedingungen zu schaffen, kann die Walzenoberfläche aber auch mit anderen oder in Längs- und Querrichtung unterschiedlichen Texturen wie Mattstellen, Prägungen, unterschiedlichen Glanz-Mattgraden, Mustern und dergleichen ausgeformt sein.

Ziel dieser Strukturen ist beispielsweise die Oberfläche z.B. für nachfolgende Untersuchungen, wie olfaktorische Untersuchungen (Gerüche), oder optische Untersuchungen vorzubereiten.

Die so in der Vorrichtung hergestellte Folie wird einer optischen Überprüfung zugeführt.

Dazu werden mittels einer hoch auflösenden Flächenkamera oder Zeilenkamera und nachgeschaltetem Hochleistungsrechner Einschlüsse und Gelpartikel detektiert und anhand einer geeigneten Auswertesoftware, welche die Größe, Form und Lage der Einschlüsse und Fehlstellen erkennt und in Klassen zuordnen kann ausgewertet.

Die Erkennung der Fehler erfolgt dabei über Grauwert- oder Farbänderungen einzelner Pixel des aufgenommenen Bildes.

Die Größe der Einschlüsse und Fehlstellen kann im einfachsten Fall über die Anzahl der eingenommenen Pixel definiert werden, kann aber auch über die Fläche des umschließenden Kreises oder umschließenden Rechtecks der beiden Extremwerte (Länge, Breite) ausgedrückt werden.

Im letzteren Fall lässt sich auch eine Aussage über die Form der Fehlstelle treffen, da es sich dabei um das Aspektverhältnis des Fehlers handelt.

Die Beleuchtung der Folie erfolgt zweckmäßigerweise über ein Domlicht und zwei parallel oberhalb und unterhalb der Folie angeordnete Ringlichter um sowohl im Reflexionsmodus, als auch im Transmissionsmodus messen zu können. Es können aber auch, Linien-, Balken- oder Flächenbeleuchtungen eingesetzt werden.

Die Beleuchtungseinheiten sind in einer leicht zu demontierenden Kapselung angebracht, um Streulicht von außen zu verhindern.

Üblicherweise wird zur Beleuchtung Weißlicht verwendet, je nach Kamera und Beleuchtungstyp können aber auch unterschiedliche Farben oder Kombinationen aus Farben im Auf- und Durchlicht verwendet werden.

Außerdem können Fehler durch Beleuchtungseinheiten, die in unterschiedlichen Winkel zur Folienoberfläche angeordnet sind, helfen die Fehlererkennung zu erleichtern.

Weiters können Filter wie linear oder zirkular polarisierende Filter vor der Kamera und/oder hinter der Kamera eingesetzt werden, um zum Beispiel Fehler besser zu detektieren oder die Analyse der Materialien durch deren optische Eigenschaften zu ergänzen.

In einem besonderen Fall können auch UV Beleuchtungseinheiten (in verschiedenen Wellenlängen) verwendet werden, um z.B. zu untersuchen, ob das Material selbst oder Verunreinigungen in bestimmten Spektralbereichen fluoreszieren.

Um all diese Eigenschaften zu messen, können ein oder mehrere Beleuchtungs- und/ oder Kameraeinheiten parallel oder in Serie kombiniert werden.

Ferner wird die Kunststofffolie einer Farbmessung unterzogen. Dabei wird die Folie mit einer Tageslichtlampe oder hintereinander mit unterschiedlichen Farben beleuchtet und das von der Folie ausgehende Licht in verschiedene Wellenlängenbereiche aufgespalten. Mittels eines Sensors oder eines Sensorsystems werden die farbmetrischen Daten errechnet.

Die Darstellung des ermittelten Farbwertes erfolgt bevorzugt im, dem Fachmann bekannten, L*a*b Farbraum. Es können aber auch andere Farbräume wie der RGB-, CMYK- oder der Pantone-Farbraum herangezogen werden.

In einer weiteren Stufe der Überprüfung wird die Folie einer NIR-Messung unterzogen. Dieses Verfahren ermöglicht es Fremdmaterialien in der Kunststofffolie zu erkennen. Dies ist insbesondere bei Recyclingkunststoffen wichtig, um eine gleichbleibende Produktqualität zu gewährleisten.

Dabei wird zweckmäßigerweise ein Multispektralsensor eingesetzt, der im Gegensatz zu herkömmlichen NIR-Spektrometern sehr kostengünstig ist.

Der Sensor arbeitet in ausgewählten Lichtwellenbereichen und misst die Absorption des Lichtes in diesem schmalen Wellenbereich. Über die Absorptionsrate kann dabei auf z.B. Verunreinigungen oder Änderungen in der Zusammensetzung geschlossen werden. Da der Sensor nur in einem schmalen Lichtspektrum arbeitet, wird dieser vorab speziell auf das zu untersuchende Material kalibriert.

Vor der mechanischen Untersuchung wird die Folie auf eine definierte Breite geschnitten.

Der verbleibende Folienstreifen wird direkt online, zum Beispiel rein mechanisch über einen Tastkopf oder über optische (z.B. IR) oder kapazitiv oder durch Strahlung (Röntgenstrahlung) auf seine Dicke vermessen und anschließend über zwei Walzenpaare verstreckt bzw. gedehnt. Das für die Verstreckung notwendige Drehmoment wird gemessen und daraus ein Äquivalent zum Elastizitätmodul (E-Modul) berechnet.

Die Verstreckung kann dabei über zwei Walzenpaare, die mit unterschiedlicher Geschwindigkeit laufen, oder über eine Kombination von zwei Walzenpaaren, die gleichlaufen, und einer dazwischen angeordneten Streckwalze, die den Bahnweg während einer Messung verändert, ausgeführt werden.

Wird das zweite Rollenpaar in eine beschleunigte Bewegung versetzt, so kann auch ein einaxialer Zugversuch mit stetig steigender Dehnung, wie er z.B. in einem Laborversuch durchgeführt wird, nachgestellt werden.

Gegebenenfalls können ergänzend alle gängigen optischen Meßverfahren in der erfindungsgemäßen Vorrichtung eingesetzt werden, wie beispielsweise Glanzmessung, Hazemessung, UV-VIS, Ellipsometrie, Röntgenfluoreszenz und dergleichen.

Es können aber auch kombinierte Analysen, wie z. B. Kratzmessung mit optischer Erkennung des Defektes, oder weitere mechanische Kennwerte wie Weiterreißversuch, Einschnürungsverhalten oder temperaturabhängige Zugeigenschaften in der erfindungsgemäßen Vorrichtung vorgesehen sein.

Besonders wichtig für den Recyclingbereich ist auch eine Prüfung auf Gerüche. Da bisher leider noch keine wirklichen Geruchsensoren existieren, kann in einem Modul aber über einen einfachen Gaschromatographen eine einfache Messung realisiert werden.

Dazu wird entweder gleich am besten bei der Herstellung der Folie also beim Gießprozess oder später die Folie noch einmal stark erhitzt, beispielsweise: durch Infrarot, Heißluft oder über eine Kontaktwalze, und die entstehenden Dämpfe abgesaugt und in einen Gaschromatographen geleitet werden.

Bei Verwendung einer sehr kurzen Messsäule beispielsweise 10 cm kann eine rasche Messung realisiert werden, um so eine Detektion verschiedener Inhaltsstoffe, die geruchsbildend oder auch neutral sein können, zu ermöglichen. Dabei gilt, je länger die Säule umso besser die Auflösung umso länger die Messzeit.

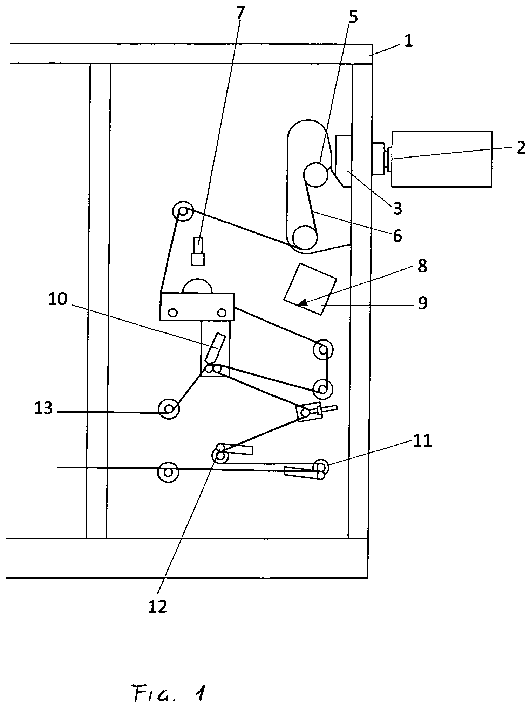

In Fig. 1 ist eine erfindungsgemäße Vorrichtung dargestellt.

Die Vorrichtung besteht aus einem Gehäuse 1, das eine Einrichtung 2 zur Entnahme der Kunststoffschmelze aufweist, die durch eine Messdüse für die Bestimmung der rheologischen Eigenschaften, insbesondere der Viskosität der Schmelze in das Innere des Gehäuses der Vorrichtung transportiert wird .

Abschließend wird die Kunststoffschmelze durch die Breitschlitzdüse 3 und eine Gießwalze 5 zu einer Flachfolie 6 geformt und über Umlenkwalzen zur ersten optischen Prüfung mittels einer Flächenkamera 7 zur Erkennung von Einschlüssen und Gelpartikeln geführt. Die Folie wird dabei durch ein Gehäuse geleitet um Streulicht auszuschließen und mit einem Domlicht und Ringlichtern beleuchtet.

Anschließend wird die Flachfolie weiter zur Farbmessung 8 und NIR-Messung 9 mittels eines Multispektralsensors geführt.

Vor der anschließenden Untersuchung wird die Folie durch einen Randbeschnitt 10 in Streifen geschnitten.

Der mittlere Streifen hat vorzugsweise eine Breite zwischen 20 und 25 mm, um somit den Normprüfkörper aus dem Folienzugversuch (ISO 527-3) zu entsprechen, kann aber auch breiter oder schmäler ausgeführt werden.

Der mittlere Streifen wird zwischen 2 Walzenpaaren 11, 12 verstreckt, um den Elastizitätsmodul zu ermitteln. Die beiden Randstreifen 13 werden nicht für den Zugversuch herangezogen.

Alle Messgeräte und die Verarbeitungseinrichtungen sind im Gehäuse platzsparend situiert.

Dadurch können die erforderlichen Parameter des Kunststoffs bzw. der Kunststofffolie direkt noch während der Produktion an einem Ort ermittelt werden und gegebenenfalls in den Produktionsprozess eingegriffen werden. Dadurch wird die Menge an nicht den Qualitätskriterien entsprechendem Material deutlich reduziert.

Durch die einmalige Probenentnahme an einer einzigen Stelle des Produktionsprozesses sind zudem keine Messstellen, die an unterschiedlichen Orten entlang der Produktionsvorrichtung situiert sind, notwendig.

## Patentansprüche

1. Vorrichtung zur Inline-Qualitätsüberprüfung für Kunststoffe während des Produktionsprozesses umfassend mindestens zwei unterschiedliche Messgeräte ausgewählt aus der Gruppe Messgeräte zur Bestimmung der rheologischen Eigenschaften, Messgeräte zur Farbmessung, Messgeräte zur Detektion von Einschlüssen und Gelpartikeln und dergleichen, Messgeräte zur Beurteilung der optischen Eigenschaften und Messgeräte zur Bestimmung der mechanischen Parameter, Untersuchung der olfaktorischen Parameter, wobei die Vorrichtung
eine Einrichtung zur Entnahme von Kunststoffschmelze aus dem Produktionsextruder,
eine Einrichtung zum Formen einer Flachfolie aus dieser Kunststoffschmelze und
Einrichtungen zum Transport der Flachfolie zu den Messgeräten und zum Konfektionieren der Flachfolie aufweist,
wobei die Vorrichtung über einen Adapter an einen Seitenstrang des Extruders gekoppelt ist und nach Öffnen eines Ventils die Entnahme der Kunststoffschmelze erfolgt, und
die Entnahme der Kunststoffschmelze durch einen Verdrängungskörper, der im Schmelzstrom positioniert ist und um den die Schmelze zu einer Bohrung im Zylindergehäuse des Extruders geleitet wird, erfolgt.

2. Vorrichtung nach Anspruch 1, **dadurch gekennzeichnet, dass** die Entnahme der Kunststoffschmelze durch eine Bohrung im Zylindergehäuse des Ausgangsbereichs des Extruders erfolgt.

3. Vorrichtung nach einem der Ansprüche 1 bis 2, **dadurch gekennzeichnet, dass** die Vorrichtung eine Breitschlitzdüse und eine Gießwalze zum Formen einer Flachfolie umfasst.

4. Vorrichtung nach einem der Ansprüche 1 bis 3, **dadurch gekennzeichnet, dass** die Vorrichtung zwei Walzenpaare zur Verstreckung der Flachfolie umfasst.

5. Vorrichtung nach einem der Ansprüche 1 bis 4, **dadurch gekennzeichnet, dass** die Vorrichtung eine Messdüse zur Bestimmung der rheologischen Eigenschaften umfasst.

6. Vorrichtung nach einem der Ansprüche 1 bis 5, **dadurch gekennzeichnet, dass** die Vorrichtung eine Flächenkamera zur Erkennung von Einschlüssen und Gelpartikeln in der Flachfolie umfasst.

7. Vorrichtung nach einem der Ansprüche 1 bis 6, **dadurch gekennzeichnet, dass** die Vorrichtung einen Multispektralsensor zur NIR Messung umfasst.

8. Vorrichtung nach einem der Ansprüche 1 bis 7, **dadurch gekennzeichnet, dass** die Vorrichtung eine Beleuchtungseinrichtung und einen Sensor zur Farbmessung umfasst.

9. Verfahren zur Qualitätsüberprüfung für Kunststoffe durch eine Vorrichtung während des Produktionsprozesses, wobei die Vorrichtung über einen Adapter an einen Seitenstrang eines Produktionsextruder gekoppelt ist, wobei das Verfahren umfasst:
Öffnen eines Ventils,
Entnehmen einer definierten Menge an Kunststoffschmelze aus dem Produktionsextruder durch einen Verdrängungskörper, der im Schmelzstrom positioniert ist und um den die Schmelze zu einer Bohrung im Zylindergehäuse des Extruders geleitet wird,
Formen dieser Kunststoffschmelze zu einer Flachfolie,
Transportieren der Flachfolie zu in der Vorrichtung situierten Messgeräten, und
Untersuchen und Beurteilen der Flachfolie.

10. Verfahren nach Anspruch 9, **dadurch gekennzeichnet, dass** das Entnehmen der Kunststoffschmelze durch eine Bohrung im Zylindergehäuse des Ausgangsbereichs des Extruders erfolgt.

11. Verfahren nach einem der Ansprüche 9 bis 10, weiterhin umfassend eines oder mehrere aus:
Formen einer Flachfolie umfasst durch eine Breitschlitzdüse und eine Gießwalze,
Verstrecken der Flachfolie durch zwei Walzenpaare,
Bestimmen der rheologischen Eigenschaften durch eine Messdüse,
Erkennen von Einschlüssen und Gelpartikeln in der Flachfolie durch eine Flächenkamera,
NIR Messung durch einen Multispektralsensor, und
Farbmessung durch eine Beleuchtungseinrichtung und einen Sensor.

## Claims

1. A device for in-line quality control for plastic materials during the production process, comprising at least two different measuring instruments selected from the group of measuring instruments for determining the rheological properties, measuring instruments for color measurement, measuring instruments for detecting inclusions and gel particles and the like, measuring instruments for assessing the optical properties and measuring instruments for determining the mechanical parameters, examination of the olfactory parameters, wherein the device has
an installation for removing plastic melt from the production extruder,
an installation for forming a flat film from this plastic melt, and
installations for transporting the flat film to the measuring devices and for finishing the flat film,
wherein the device is coupled to a side branch of the extruder via an adapter and the removal of the plastic melt is performed after opening a valve, and
the removal of the plastic melt is performed by means of a displacement body which is positioned in the melt flow and around which the melt is directed to a bore in the cylinder housing of the extruder.

2. The device according to claim 1, **characterized in that** the removal of the plastic melt is performed through a bore in the cylinder housing of the exit region of the extruder.

3. The device according to one of claims 1 to 2, **characterized in that** the device comprises a wide slit die and a casting roller for forming a flat film.

4. The device according to one of claims 1 to 3, **characterized in that** the device comprises two pairs of rollers for stretching the flat film.

5. The device according to one of claims 1 to 4, **characterized in that** the device comprises a measuring die for determining the rheological properties.

6. The device according to one of claims 1 to 5, **characterized in that** the device comprises an area scan camera for detecting inclusions and gel particles in the flat film.

7. The device according to one of claims 1 to 6, **characterized in that** the device comprises a multispectral sensor for NIR measurement.

8. The device according to one of claims 1 to 7, **characterized in that** the device comprises an illumination device and a sensor for color measurement.

9. A method for the quality control of plastic materials by means of a device during the production process, wherein the device is coupled via an adapter to a side branch of a production extruder, wherein the method comprises:
opening a valve,
removing a defined amount of plastic melt from the production extruder by means of a displacement body which is positioned in the melt flow and around which the melt is directed to a bore in the cylinder housing of the extruder,
forming this plastic melt into a flat film,
transporting the flat film to measuring devices situated in the device, and
examining and evaluating the flat film.

10. The method according to claim 9, **characterized in that** the removal of the plastic melt is performed through a bore in the cylinder housing of the exit region of the extruder.

11. The method according to one of claims 9 to 10, further comprising one or more of:
forming a flat film by means of a wide slit die and a casting roller,
stretching the flat film using two pairs of rollers,
determining the rheological properties using a measuring nozzle,
detecting inclusions and gel particles in the flat film using an area scan camera, NIR measurement using a multispectral sensor, and
color measurement using an illumination device and a sensor.

## Revendications

1. Dispositif pour le contrôle qualité en ligne de matières plastiques pendant le processus de fabrication, comprenant au moins deux appareils de mesure différents sélectionnés dans le groupe constitué d'appareils de mesure pour la détermination des propriétés rhéologiques, d'appareils de mesure pour la mesure des couleurs, d'appareils de mesure pour la détection d'inclusions et de particules de gel et autres, d'appareils de mesure pour l'évaluation des propriétés optiques et d'appareils de mesure pour la détermination des paramètres mécaniques, l'examen des paramètres olfactifs,
un dispositif pour le prélèvement de matière plastique fondue dans l'extrudeuse de production,
un dispositif pour le formage d'un film plat à partir de cette matière plastique fondue et
des dispositifs pour le transport du film plat vers les appareils de mesure et pour la confection du film plat,
dans lequel le dispositif est couplé, par l'intermédiaire d'un adaptateur, à une branche latérale de l'extrudeuse et le prélèvement de la matière plastique fondue a lieu après l'ouverture d'une soupape et
le prélèvement de la matière plastique fondue a lieu à l'aide d'un corps de refoulement qui est positionné dans le flux de fonte et autour duquel la fonte est guidée vers un alésage dans le boîtier cylindrique de l'extrudeuse.

2. Dispositif selon la revendication 1, **caractérisé en ce que** le prélèvement de la matière plastique fondue a lieu à travers un alésage dans le boîtier cylindrique de la zone de sortie de l'extrudeuse.

3. Dispositif selon l'une des revendications 1 à 2, **caractérisé en ce que** le dispositif comprend une buse à large fente et un cylindre de coulée pour le formage d'un film plat.

4. Dispositif selon l'une des revendications 1 à 3, **caractérisé en ce que** le dispositif comprend deux paires de cylindres pour l'étirage du film plat.

5. Dispositif selon l'une des revendications 1 à 4, **caractérisé en ce que** le dispositif comprend une buse de mesure pour la détermination des propriétés rhéologiques.

6. Dispositif selon l'une des revendications 1 à 5, **caractérisé en ce que** le dispositif comprend une caméra de surface pour la détection d'inclusions et de particules de gel dans le film plat.

7. Dispositif selon l'une des revendications 1 à 6, **caractérisé en ce que** le dispositif comprend un capteur multi-spectral pour une mesure NIR.

8. Dispositif selon l'une des revendications 1 à 7, **caractérisé en ce que** le dispositif comprend un dispositif d'éclairage et un capteur pour la mesure des couleurs.

9. Procédé de contrôle qualité pour des matières plastiques à l'aide d'un dispositif pendant le processus de production, dans lequel le dispositif est couplé, par l'intermédiaire d'un adaptateur, à une branche latérale d'une extrudeuse de production, dans lequel le procédé comprend :
l'ouverture d'une soupape,
le prélèvement d'une quantité définie de matière plastique fondue dans l'extrudeuse de production à l'aide d'un corps de refoulement, qui est positionné dans le flux de fonte et autour duquel la fonte est guidée vers un alésage dans le boîtier cylindrique de l'extrudeuse,
formage de cette matière plastique fondue en un film plat,
transport du film plat vers des appareils de mesure situés dans le dispositif et
examen et évaluation du film plat.

10. Procédé selon la revendication 9, **caractérisé en ce que** le prélèvement de la matière plastique fondue a lieu à travers un alésage dans le boîtier cylindrique de la zone de sortie de l'extrudeuse.

11. Procédé selon l'une des revendications 9 à 10, comprenant en outre une ou plusieurs des étapes suivantes :
formage d'un film plat à l'aide d'une buse à large fente et d'un cylindre de coulée,
étirage du film plat à l'aide de deux paires de cylindres,
détermination des propriétés rhéologiques à l'aide d'une buse de mesure,
détection d'inclusions et de particules de gel dans le film plat à l'aide d'une caméra de surface,
mesure NIR à l'aide d'un capteur multi-spectral et
mesure de couleur à l'aide d'un dispositif d'éclairage et d'un capteur.
